# EUROPEAN PATENT APPLICATION

(11) **EP 1 543 834 A1**
(43) Date of publication of application: **22.06.2005**
(21) Application number: 03447290.2
(22) Date of filing: 16.12.2003
(51) Int. Cl.: A61K 35/78, A61P 35/00, A61P 15/12, A61P 19/10, A61P 25/28, A61P 9/10, A61P 29/00, A61P 5/48, A61P 5/24

(54) **Production of hop extracts having oestrogenic and antiproliferative bioactivity**

(71) Applicant: Biodynamics, 8400 Oostende (BE)
(72) Inventor: De Keukeleire, Denis, Lab.of Pharm. and Phytochem., 9000 Ghent (BE); Heyerick, Arne, Lab. of Pharm. and Phytochem., 9000 Ghent (BE); Maes, Francis, 8400 Oostende (BE)
(74) Representative: Luys, Marie-José

(57) **Abstract**

The invention relates to a method for the production of a hop extract enriched in prenylated flavonoids or derivatives thereof, whereby the method comprises the steps of subjecting a hop product to an isomerisation reaction in the presence of an amount of a base and to at least one extraction.

## Description

The present invention relates to a method for the production of hop extracts enriched in prenylated flavonoids and derivatives thereof having combined oestrogenic and antiproliferative bioactivities.

Hops contains three major classes of secondary metabolites namely the hop (bitter) acids, the hop essential oil, and the hop polyphenols. Hop acids and the hop essential oil, and to a certain amount low-molecular-weight polyphenols, are the most important hop constituents for the purpose of beer brewing. Hop extracts for brewing purposes are generally prepared by extracting hops or hop products with liquid or supercritical CO₂ in view of obtaining hop extracts containing the above mentioned secondary metabolites in the desired proportions.

Recently, significant research has been focused on the biological activity of prenylated flavonoids, which constitute a specific class of polyphenols. The most important prenylated flavonoids present in fresh hops are *chalcones,* in particular xanthohumol and desmethylxanthohumol. These chalcones are precursors for *flavanones* such as isoxanthohumol (which originates from xanthohumol), 8-prenylnaringenin and 6-prenylnaringenin (which both originate from desmethylxanthohumol) (see figure 1). 8-Prenylnaringenin has been identified as the active principle of the oestrogenic activity of hops with an activity greater than that of other established phyto-oestrogens (Milligan et al., 1999). Next to 8-prenylnaringenin, other similar hop-derived compounds, such as 6-prenylnaringenin, isoxanthohumol, 6,8-diprenylnaringenin, and 8-geranylnaringenin (Milligan et al., 2000) were found to be only weakly oestrogenic. The high oestrogenic activity of 8-prenylnaringenin was confirmed by *in vivo* tests (Milligan et al., 2002).

Xanthohumol on the other hand has been shown to be a very potent cancer chemopreventive compound *in vitro* with an exceptional broad spectrum of inhibitory mechanisms at the initiation, promotion, and progression stages of carcinogenesis (Gerhauser et al., 2002). Consistent with the anti-initiating potential, xanthohumol potently modulates the activity of enzymes involved in carcinogen metabolism and detoxification, e.g. CYP450-enzymes (Henderson et al., 2000; Miranda et al., 2000c) and quinone reductases (Miranda et al., 2000a). Moreover, xanthohumol has been found capable of scavenging reactive oxygen species including hydroxyl and peroxyl radicals (Miranda et al., 2000b; Rodriguez et al., 2001) and of inhibiting superoxide anion radical and nitric oxide production (Zhao et al., 2003). As potential anti-tumour promoting activity, xanthohumol demonstrates anti-inflammatory properties by inhibition of cyclooxygenase-1 and cyclooxygenase-2 activity (Gerhauser et al., 2002). Antiproliferative mechanisms to prevent carcinogenesis in the progression phase include inhibition of DNA synthesis and induction of cell cycle arrest in the S-phase, apoptosis, and cell differentiation (Miranda et al., 1999; Gerhauser et al., 2002). Furthermore, xanthohumol proved efficient at nanomolar concentrations in preventing carcinogen-induced preneoplastic lesions in mouse mammary gland organ culture, a model which serves as a link between short-term *in vitro* and longterm *in vivo* carcinogenesis models (Gerhauser et al., 2002).

The above described flavonoids are non-essential products in the process of beer brewing. They largely remain behind in the residue left after extraction of the desired compounds from hops with liquid or supercritical CO₂. In normal brewing conditions, whereby either whole hops, hop products, or particular hop fractions are used, the total concentration of prenylated flavonoids may be up to 4 mg per litre of beer (Stevens et al., 1999).

In DE 199 39 350, a method for the production of a hop extract enriched in xanthohumol is described, according to which a hop product is extracted with an organic solvent or alkaline water. Prior to this extraction, the hop product may be extracted with water in view of removing hydrophilic substances. As a suitable organic solvent, a water/ethanol solution is disclosed. Nothing however is mentioned about the presence of prenylated flavanones in the hop extract.

WO03014287 discloses that the oestrogenic activity of hops extracts must be primarily attributed to the presence of 8-prenylnaringenin, which has been found to be the compound showing the most important oestrogenic activity. 6-prenylnaringenin also shows oestrogenic activity, although lower than the 8-prenylnaringenin. It is reported that xanthohumol is capable of interfering in cell metabolism and can be regarded as a cancer preventing agent. The hop extracts of WO03014287 are disclosed to be suitable for use in the prophylaxis or therapy of disease states caused by a lack of oestrogens or disturbances in the metabolism of sex steroidal hormones, in particular of oestrogens.

WO03014287 also discloses a method for producing a hop extract, which is enriched in chalcones and flavanones, such as xanthohumol, isoxanthohumol, 6-prenylnaringenin, and 8-prenylnaringenin. The method comprises the steps of
(1) extracting hops or a hop product with a C₅-C₇ alkane or supercritical CO₂ for removing hydrophobic substances contained in hops,
(2) extracting the residue obtained in step (1) with water in order to remove hydrophilic substances contained in hops, and, finally,
(3) extracting the residue obtained in step (2) with an organic solvent selected from the group consisting of alcohols, water-based alcohols, ketones, water-based ketones or esters or mixtures thereof.
   The concentration of isoxanthohumol, 6-prenylnaringenin and 8-prenylnaringenin in the thus obtained hop extract have been found to increase with an increasing temperature during water extraction in step (2).

The hop extract disclosed in WO02/08539 is reported to contain at least 3 wt. % of prenylated flavonoids, in particular xanthohumol, isoxanthohumol, and 8-prenylnaringenin. When comparing the oestrogenic activity of the hop extracts disclosed in WO02/085393, a plurality of the extracts did not show enhanced oestrogenic activity when compared to a traditional, non-enriched extract. Moreover, no correlation between the composition of the extract and oestrogenic activity was shown. Also, no oestrogenicity was attributed to 8-prenylnaringenin. It is further alleged that the oestrogenic properties of the extract would be particularly expressed when containing 1-30 wt. % of xanthohumol, 0.01-50 wt. % of isoxanthohumol, 0.0005-10 wt. % of 8-prenylnaringenin. The hop extract of WO02/085393 is said to be suitable for manufacturing medicaments having oestrogenic properties, cosmetic compositions, nutritional supplements, and dietary preparations.

Although methods have been developed for enriching hop extracts in prenylated flavonoids, methods to obtain specific compositions of relevant prenylated flavonoids in hop extracts are not characterised.

It is therefore an object of the present invention to provide a method for the production of hop extracts having specific compositions of relevant prenylated flavonoids or derivatives thereof with respect to their biological activities.

This is achieved with the present invention by means of a process showing the technical features of the characterising part of the first claim.

The method of the present invention is characterised in that hops or a hop product is dissolved in a solvent and subjected to (1) an isomerisation reaction in the presence of an amount of a base and (2) at least one extraction.

Hops or a hop product is understood to comprise any form of processed hops, e.g., pelletized hops or any hop extract or any hop-derived residue containing prenylated flavonoids.

The inventor has now found that with this method a hop extract can be obtained which is further enriched in prenylated flavonoids, especially in 8-prenylnaringenin and xanthohumol, as compared to the state of the art. With the process of this invention a hop extract may be obtained which shows a higher oestrogenic activity due to the enrichment in 8-prenylnaringenin. Furthermore, the extract has been found to show improved proliferative activity inhibiting properties due to the enrichment in xanthohumol as compared to the state of the art compositions, proliferative activity being due to the presence of 8-prenylnaringenin (see figure 2). In this respect is important to note that xanthohumol shows an exceptionally broad spectrum of inhibition mechanisms at all stages of carcinogenesis, i.e., initiation, promotion, and progression (Gerhauser et al., 2002).

The sequence, in which the isomerisation and the at least one extraction are carried out, is not critical to the invention. In practise this means that either one of the isomerisation or the extraction may be carried out first.

### 1. The isomerisation reaction.

It is known that, at elevated temperatures, both desmethylxanthohumol and xanthohumol - the most important prenylated flavonoids (chalcones) present in hops or a hop product - undergo facile thermal isomerisation through Michael-type intramolecular cycloaddition into their corresponding flavanones. While xanthohumol is virtually exclusively isomerised to isoxanthohumol, isomerisation of desmethylxanthohumol leads to formation of a mixture of 8-prenylnaringenin (8-PN) and 6-prenylnaringenin (6-PN), in a ratio of (8-PN x 100%)/(8-PN + 6-PN), which usually is between 15% and 25%. With the present invention it has surprisingly been found that, by carrying out the isomerisation reaction in specific alkaline conditions, the ratio (8-PN x 100%)/(8-PN + 6-PN) may be shifted in favour of 8-PN, the total yield of (8-PN + 6-PN) remaining virtually unaffected.

As 8-PN is the compound having the higher oestrogenic activity when compared to 6-prenylnaringenin or any other hop-derived compound, the oestrogenic activity of the thus obtained hop extract may be increased 3-5 times as compared to the known hop extracts (e.g., hydroalcoholic extraction at elevated temperatures).

A preferred embodiment of the method of this invention is characterised in that the isomerisation reaction is carried out in alkaline conditions corresponding to concentrations of KOH in water (w/v %) of at least 0.5, preferably of at least 1, more preferably of at least 5.

Carrying out the isomerisation reaction in increasing alkaline conditions allows to shift the ratio (8-PN x 100%)/(8-PN + 6-PN) from 15-25 %, as known from the state of the art, to 60 or 75 % or even higher in favour of 8-PN, while the total yield of (8-PN + 6-PN) remains virtually unaffected. As a result, with the method of the present invention a hop extract may be obtained which is highly enriched in 8-PN. Or in other words, with the present invention the ratio 6-PN/8-PN may be reversed and, whereas with the state-of-the-art techniques a selective enrichment in 6-PN was obtained, the present invention provides a selective enrichment in 8-PN, the compound having the higher oestrogenic activity.

To minimise the risk to oxidation of the reactants or reaction products, the isomerisation reaction is preferably carried out in inert atmosphere. The reactants, in particular xanthohumol and desmethylxanthohumol, as well as the reaction products isoxanthohumol and 8-prenylnaringenin contain double bonds and phenolic groups, which are susceptible to oxidation.

The temperature at which the isomerisation reaction is carried out is not critical to the invention, and may be any temperature between 0°C and the boiling point of the reaction mixture, although a temperature of between 40 and 60°C, in particular about 50°C is preferred. In this preferred range namely an optimum selectivity towards isoxanthohumol and 8-prenylnaringenin has been found, formation of undesired side products being reduced to a minimum, reaction rate being optimum.

The inventors have further found that the isomerisation reaction proceeds at a high reaction rate, and may virtually be complete within 15 minutes, if carried out at the optimum reaction temperature. It is preferred to carry out the isomerisation reaction for a time period between 0.25 and 2 h. The person skilled in the art will, in general, be capable of adjusting the duration and the temperature at which the isomerisation reaction is carried out, to obtain optimum conversion and selectivity towards the desired end products, and to minimise the risk to the formation of unwanted side products if the reaction is continued for a too long period of time.

### 2. The extraction process.

To isolate the desired compounds from the hops or hops product either before of after having subjected the hops or hop product to an isomerisation reaction, the hops or hop product is subjected to at least one extraction.

Extraction of the prenylated flavonoids is obtained by subjecting the hops or hop product to an extraction with at least one organic solvent chosen from the group of alcohols, water-based alcohols, ketones, water-based ketones or esters or mixtures thereof or alkaline water. Examples of organic solvents suitable for use are solvents of medium polarity, e.g., ethyl acetate or acetone, or high polarity, e.g., ethanol or methanol, or mixtures thereof such as a mixture consisting of 90/10 (v/v) ethyl acetate/methanol or a hydroalcoholic mixture (75/25 (v/v) ethanol/water).

Preferably, before or after subjecting the hops or the hop product to the at least one extraction or before or after subjecting the hops or the hop product to the isomerisation reaction, the hops or the hop product is subjected to an additional extraction step with water and/or at least one non-polar organic solvent, followed by recovering the residue or the extract enriched in prenylated flavonoids. An additional extraction with water allows to remove non-functional hydrophilic ballast material such as proteins, thus leading to a higher enrichment of prenylated flavonoids in the obtained hop extract, whereas an additional extraction with at least one non-polar organic solvent allows to further remove non-functional hydrophobic (lipophilic) ballast material, thus further leading to a higher enrichment or concentration of prenylated flavonoids in the residue or the extract enriched in prenylated flavonoids.

In case the concentration of hydrophobic ballast material in the hops or a hop product starting material is too high, an additional extraction with at least one non-polar organic solvent may be envisaged. The product subjected to such an additional extraction will mostly be the hop residue remaining after the primary extraction of hops with liquid or supercritical CO₂ performed in non-exhaustive conditions (mild temperature and pressure) to separate the compounds of interest for beer brewing from the residue containing more polar secondary metabolites including relevant prenylated flavonoids.

The extraction of hydrophilic and hydrophobic ballast material can be carried out by means of solvent-solvent extractions (e.g. water vs. ethyl acetate, hexane vs. hydroalcoholic mixtures) or solid-phase extractions (solubilisation of hydrophilic and hydrophobic material in water and a non-polar organic solvent, respectively, or use of silica or derivatised silica) or in any other suitable way known to the person skilled in the art. Separation of the fraction with prenylated flavonoids after extractions with water and the non-polar organic solvent, respectively, can, e.g., be carried out by means of decantation, filtration, and/or centrifugation.

A possible extraction method, for example, involves extracting hops with fluid or supercritical CO₂ and recovering the obtained residue, followed by subjecting the residue to the isomerisation reaction in alkaline conditions. Subsequently, the reaction mixture containing the boiled spent hops is acidified until neutral pH and the residue is recovered, e.g., by filtration or centrifugation after washing with water. The prenylated flavonoids can then be extracted selectively from the residue by using suitable solvents or mixtures thereof.

### 3. Pre-processing

In view of improving the economical feasibility of the present invention and further concentrating the desired compounds, the hops or hop product is subjected to an extraction in liquid or supercritical CO₂ or at least one substantially non-polar organic solvent, followed by recovering the residue containing the prenylated flavonoids, before subjecting it to the combined isomerisation reaction and extraction.

This means in fact that the extract of interest for brewing purposes is recovered, while the residue is enriched in prenylated flavonoids and deprived of a major part of hydrophobic ballast material through pre-extraction. This pre-enriched residue is then subjected to the isomerisation and the at least one extraction in arbitrary order.

### 4. Appropriate formulation

The inventors have found that possible proliferation induced by the oestrogenic activity of 8-prenylnaringenin can be inhibited by xanthohumol. However, such inhibition can only be obtained provided the weight ratio of xanthohumol to 8-prenylnaringenin is at least 10, preferably be at least 30. This can be achieved by addition of xanthohumol in excess (see figure 2). In other words, at particular weight ratios of xanthohumol to 8-prenylnaringenin, an efficient inhibition of the proliferative activity associated with the oestrogenicity of 8-prenylnaringenin can be obtained by the antiproliferative activity of xanthohumol.

In this respect, figure 2 shows that increasing concentrations of 8-prenylnaringenin lead to increased proliferation of MCF-7 breast cancer cells. Surprisingly, for each concentration of 8-prenylnaringenin, low concentrations of xanthohumol (< 1 µM) stimulated the proliferation induced by 8-prenylnaringenin, whereas concentrations of xanthohumol of at least 5 µM significantly inhibited the proliferation induced by 8-penylnaringenin.

Under conditions for most efficient isomerisation of desmethylxanthohumol to 8-prenylnaringenin and 6-prenylnaringenin in favour of 8-prenylnaringenin, xanthohumol is almost quantitatively isomerised to isoxanthohumol. On the other hand, a hop extract, which is enriched in xanthohumol, can be produced by subjecting hops or a hop product to at least one extraction under non-isomerising conditions (extraction under neutral or acidic conditions at room temperature).

Thus, preferably, the method of the present invention comprises the mixing of the extract, obtained by the above described method of a combined isomerisation - extraction procedure, with a second hop extract enriched in xanthohumol. This allows to obtain a hop extract with a specific weight ratio of xanthohumol to 8-prenylnaringenin. It is preferred that the weight ratio of xanthohumol to 8-prenylnaringenin is at least 10, more preferably at least 30, as, within these ranges, a mixture is obtained showing an appropriate combination of oestrogenic activity, due to 8-prenylnaringenin, and cancer-chemopreventive activity, due to xanthohumol. This mixing allows to compensate for any decrease in the xanthohumol content of the extract that had been subjected to the isomerisation reaction, because of xanthohumol isomerisation to isoxanthohumol. By mixing an extract enriched in 8-prenylnaringenin, obtained from the combined extraction - isomerisation of this invention, with an extract enriched in xanthohumol, the present invention allows to obtain a hop extract with a specific weight ratio of 8-prenylnaringenin to xanthohumol, independent of the degree of conversion of xanthohumol to isoxanthohumol in the isomerisation reaction.

Thus, with the present invention a hop extract can be obtained which contains at least 0.25 wt. % 8-prenylnaringenin and at least 2.5 wt. % xanthohumol, more preferably of at least 0.5 wt. % 8-prenylnaringenin and at least 10 wt. % xanthohumol.

A suitable method for obtaining the extract enriched in xanthohumol comprises the steps of subjecting hops or a hop product to at least one extraction by means of liquid or supercritical CO₂ or at least one substantially non-polar organic solvent, followed by recovery of the residue enriched in xanthohumol, and subjecting the residue to at least one further extraction with suitable solvents or mixtures thereof in view of further concentrating xanthohumol.

### 5. Increasing the oestrogenic potential

In a further preferred embodiment of the method for the production of a hop extract of the present invention, the method, additionally, comprises the steps of mixing the extract obtained by the at least one extraction and the isomerisation reaction with an amount of an extract enriched in a compound having high oestrogenic activity such as 8-isopentylnaringenin, which is hydrogenated 8-prenylnaringenin.

The reason for the addition of an amount of 8-isopentylnaringenin is that the inventors surprisingly found that 8-isopentylnaringenin shows oestrogenic activity and that this oestrogenic activity is only 3-4 times weaker than that of 8-prenylnaringenin (see figure 4). With respect to 8-isopentylnaringenin it is remarked that although its oestrogenic activity was found to be 3-4 times weaker than that of 8-prenylnaringenin, 8-isopentylnaringenin was found to show a higher bioavailability and better stability as compared to 8-prenylnaringenin, as it is less easy metabolised because of the absence of the double bond in the side chain.

An extract enriched in 8-isopentylnaringenin can be obtained using several methods.

A first possible method includes the steps of:
a. subjecting a hop extract enriched in xanthohumol to an isomerisation reaction to convert xanthohumol to isoxanthohumol;
b. subjecting the extract obtained in step (a) to a catalytic hydrogenation reaction to convert isoxanthohumol to dihydro-isoxanthohumol. The catalytic hydrogenation is preferably carried out by admitting H₂ in the presence of a hydrogenation catalyst such as supported Pt or Pd, or any other catalyst. An example of a suitable carrier is activated carbon. The hydrogenation reaction is preferably carried out in MeOH;
c. subjecting the reaction product of step (b) to a de-methylation reaction. This is done to convert dihydroisoxanthohumol to 8-isopentylnaringenin. The de-methylation reaction may for example be carried out using boron tribromide or any other de-methylating agent.

Thus, with the above described method the oestrogenically inactive xanthohumol may be converted into a compound having high oestrogenic activity, namely 8-isopentylnaringenin. This is a serious advantage as the concentration of xanthohumol in hops is significantly higher (0.3-1.2 wt. %) than the concentration of desmethylxanthohumol (0.005-0.2 wt. %), the precursor of 8-prenylnaringenin. With the above described method a hop extract may be obtained, which is not only enriched in 8-PN but also in 8-isopentylnaringenin, as a result of which the oestrogenic activity may be significantly increased.

A second possible method of obtaining an extract containing 8-isopentylnaringenin, is the addition of this product obtained through a synthetic way. Although substitution on the 8-position usually proceeds with low selectivity and yield because a large number of reaction steps are involved, the inventors have now developed a method for the synthesis of 8-isopentylnaringenin, which comprises only a limited number of reaction steps at high yield.

According to this method, 2,4,6-trimethoxy-benzaldehyde (see figure 3) is subjected to an alkylation reaction in the presence of an organometallic reagent (e.g., isopentyl lithium), followed by deoxygenation to 1-alkyl-2,4,6-trimethoxybenzenes (e.g. 1-isopentyl-2,4,6-trimethoxybenzene). The deoxygenation is preferably carried out in the presence of triethylsilane in trifluoroacetic acid. 1-Alkyl-2,4,6-trimethoxybenzene is subjected to an acetylation reaction followed by a de-methylation to furnish 3-alkyl-2,4,6-trihydroxyacetophenones (e.g., 3-isopentyl-2,4,6-trihydroxyacetophenone). The acetylation reaction is preferably carried out in the presence of acetyl chloride and tin tetrachloride. De-methylation is preferably carried out in the presence of boron tribromide.

In the course of this synthesis route it is preferred to selectively protect two phenolic groups as methoxymethyl ethers (MOM). The third phenol group has been found to resist reaction because of strong intra-molecular hydrogen binding.

The thus obtained de-methylated reaction product is subjected to a mixed aldol reaction involving MOM-protected benzaldehyde, to give formation of chalcones, which undergo an intramolecular Michael-type cycloaddition to the corresponding flavanones. By removing the protective groups in the acidic environment 8-alkylnaringenins (e.g., 8-isopentylnaringenin) are obtained.

### 6. Further isomerisation of isoxanthohumol

The inventors have further surprisingly found that in a system simulating the anaerobic ecology in the intestine isoxanthohumol is converted very efficiently to 8-prenylnaringenin.

This conversion is extremely important for various reasons:
- in the method of the present invention, the hops or hop product is subjected to a combined isomerisation and extraction procedure, in view of converting desmethylxanthohumol to 8-prenylnaringenin to increase the oestrogenic activity. When subjecting hops or a hop product to the combined isomerisation-extraction, simultaneously the oestrogenically inactive xanthohumol may be quantitatively isomerised into isoxanthohumol, which in the anaerobic ecology present in the intestines is converted into the oestrogenic active 8-prenylnaringenin. The method of this invention thus provides significant oestrogenic upgrading of both essential chalcones present in hops or hop products into 8-prenylnaringenin. Hence a lower daily dose of 8-prenylnaringenin may be applied;
- although isoxanthohumol is only weakly oestrogenic itself, it should in fact be considered a pro-oestrogen, in addition to desmethylxanthohumol, as it gives rise to the formation of the potent oestrogen 8-prenylnaringenin, thereby considerably enhancing the oestrogenic potential of the above described hop extracts;
- very important consequences relate to beers, containing prenylated flavonoids, since isoxanthohumol is by far the most prevalent prenylated flavonoid (> 90%), hence, the oestrogenic potential of beers could be unexpectedly high.

### 7. Conclusions

The present invention relates to a hop extract comprising a mixture of xanthohumol and 8-prenylnaringenin, whereby the ratio xanthohumol to 8-prenylnaringenin is at least 10, preferably at least 20, more preferably at least 30.

Preferably, the hop extract of the present invention comprises at least 0.25% (w/v) 8-prenylnaringenin and at least 2.5% (w/v) xanthohumol, more preferably of at least 0.5% (w/v) 8-prenylnaringenin and at least 10% xanthohumol.

Also, preferably, hop extracts of the present invention further comprise isoxanthohumol (as pro-oestrogen) and 6-prenylnaringenin. The ratio (8-prenylnaringenin x 100%)/(8-prenylnaringenin + 6-prenylnaringenin) should be at least 25%, preferably at least 50%, more preferably at least 75%.

In another preferred embodiment of the present invention, the hop extracts may contain the oestrogenically very active 8-isopentylnaringenin, derived from semi-synthesis based on a hop extract or from pure synthesis.

### 8) Indications

The hop extract of the present invention or the hop extract obtainable with the methods of the present invention can be used for the manufacture of a medicament or a phytopharmaceutical having combined oestrogenic and cancer-chemopreventive activities.

Preferably, the cancer-chemopreventive action includes anti-proliferative action.

Further, the hop extract of the present invention or the hop extract obtainable with the methods of the present invention can be used for a medicament or a phytopharmaceutical for the treatment or prophylaxis of conditions or symptoms or complaints or disease states caused by a disturbance in hormonal balance of oestrogenic nature.

Preferably, the condition or symptom or complaint or disease state caused by the disturbance in hormonal balance of oestrogenic nature is related to the menopause.

Also preferably, the disease state is osteoporosis.

In another preferred embodiment, the disease is selected from the group consisting of Alzheimer's disease, sex hormone-dependent cancers, cardiovascular diseases, diabetes, inflammatory diseases, depression, prostate dysfunction, leukemia, inflammatory bowel disease, colon cancer.

The invention further relates to a nutritional composition/supplement comprising the hop extract of the present invention or the hop extract obtainable with the methods of the present invention.

The invention also relates to a cosmetic composition comprising the hop extract of the present invention or the hop extract obtainable with the methods of the present invention.

The hop extracts resulting from the present invention can be incorporated into various formulations including pills, capsules, gelules, solutions and the likes, while the usual excipients may be applied for best usage and bioavailability.

### 9) Examples and figures

The invention is further elucidated in following examples and figures.

### Extraction

Spent hops (hop variety Nugget; 202.74 g), i.e. the residue left after extraction of natural hops with fluid or supercritical CO₂ was extracted by maceration under ambient temperature with 1 I of a 90/10 (v/v) solvent mixture of ethyl acetate and methanol in view of selectively extracting xanthumol and desmethylxanthohumol from the spent hops. The extract was filtered off and the extract enriched in xanthohumol and desmethylxanthohumol (720 ml) was recovered. After evaporating the solvent under reduced pressure, the residue was re-dissolved in 100 ml of a hexane/methanol 1/1 (v/v) mixture with the aim of extracting lipophilic ballast material and transferred to a separatory funnel. After addition of 30 ml of acidified water (1 N HCl), the hexane layer containing the lipophilic ballast material was discarded. The remaining methanolic layer was subjected to a reduced pressure to evaporate the solvent. After addition of water (70 ml) and ethyl acetate (100 ml), and phase separation, the organic layer containing the final hop extract was dried.
Yields:
- Final hop extract 4.64 g (2.3%)
- Desmethylxanthohumol 2.41 wt. % *(as measured by HPLC)*
- Xanthohumol 33.62 wt. % *(as measured by HPLC)*

### Isomerisation

The hop extract (0.5 g) was boiled for 1 h in water (40 ml) containing varying amounts of KOH (0 g, 0.2 g, 0.4 g, and 2 g, respectively). The solution was acidified (HCl until neutral) and cooled to ambient temperature. The extract was recovered by extraction with ethyl acetate and, after removal of the solvent, dried. The results of the quantitative HPLC-analyses are shown in table 1. It should be noted that the extract, resulting from boiling without the addition of a base, is very similar to the commercially available hop extracts, whereas addition of increasing amounts of base significantly increased the ratio (8-prenylnaringenin x 100%)/(8-prenylnaringenin + 6-prenylnaringenin) in favour of 8-prenylnaringenin.

**Table 1.**

| **Ratio of (8-prenylnaringenin x 100)/(8-prenylnaringenin + 6- prenylnaringenin) under varying alkaline conditions** | |
|---|---|
| KOH (w/v %) | Ratio (%) |
| 0 | 22.4 |
| 0.5 | 34.7 |
| 1 | 58.6 |
| 5 | 74.8 |

### Appropriate formulation

Figure 2 shows the growth stimulation of MCF-7 breast cancer cells by 8-prenylnaringenin and inhibition of this proliferation in the presence of increasing concentrations of xanthohumol.

A bioassay was developed to probe proliferation of oestrogen-dependent cancer cells by 8-prenylnaringenin and to determine concentrations of xanthohumol required to inhibit this 8-prenylnaringenin-induced proliferation. Thereto, oestrogen-responsive MCF-7 breast cancer cells were grown in 96-well plates in the presence of a fixed and growth-stimulatory concentration of 8-prenylnaringenin and varying concentrations of xanthohumol. For each concentration of 8-prenylnaringenin (1 nM, 10 nM, and 100 nM), the concentrations of xanthohumol were 0 µm, 0,1 µM, 1 µM, 5 µM, 10 µM and 25 µM, respectively.

### Increasing the oestrogenic potential

Figure 4 shows the dose-response curves for the oestrogenic activity of 17β-estradiol (E2), 8-prenylnaringenin (8PN), 8-isopentylnaringenin (8PN-H2), and the intermediate dihydroisoxanthohumol (IsoX-H2), as measured with an oestrogen-inducible yeast screen *(Saccharmomyces cerevisiae)* expressing the human oestrogen receptor and containing expression plasmids carrying oestrogen-responsive sequences controlling the reporter gene Lac-Z (Milligan et al., 2001). The yeasts were grown in a medium containing increasing concentrations of 17β-estradiol (positive control), 8-prenylnaringenin (8-PN), 8-isopentylnaringenin (8PN-H2) or dihydro-isoxanthohumol (IsoX-H2). Expression of the Lac-Z reporter gene was spectrophotometrically measured and quantified.

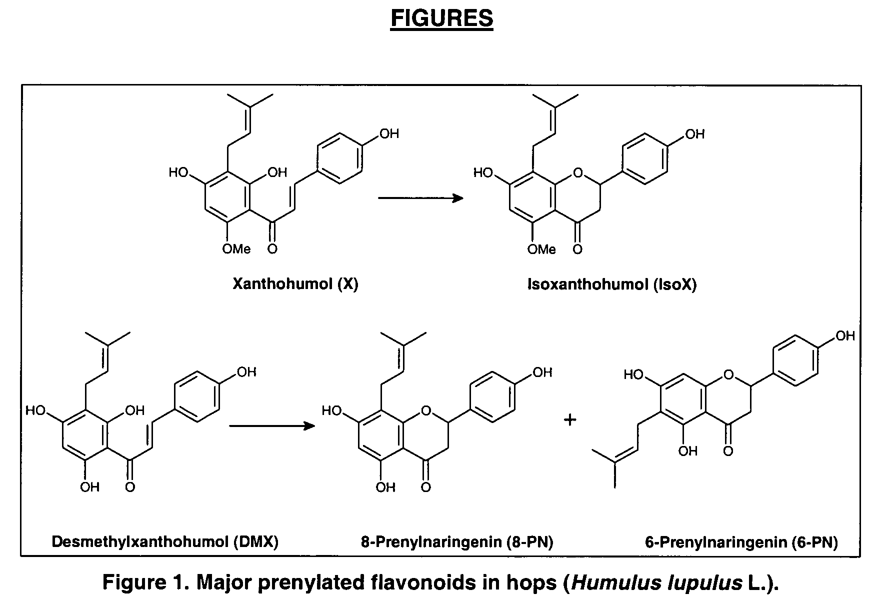

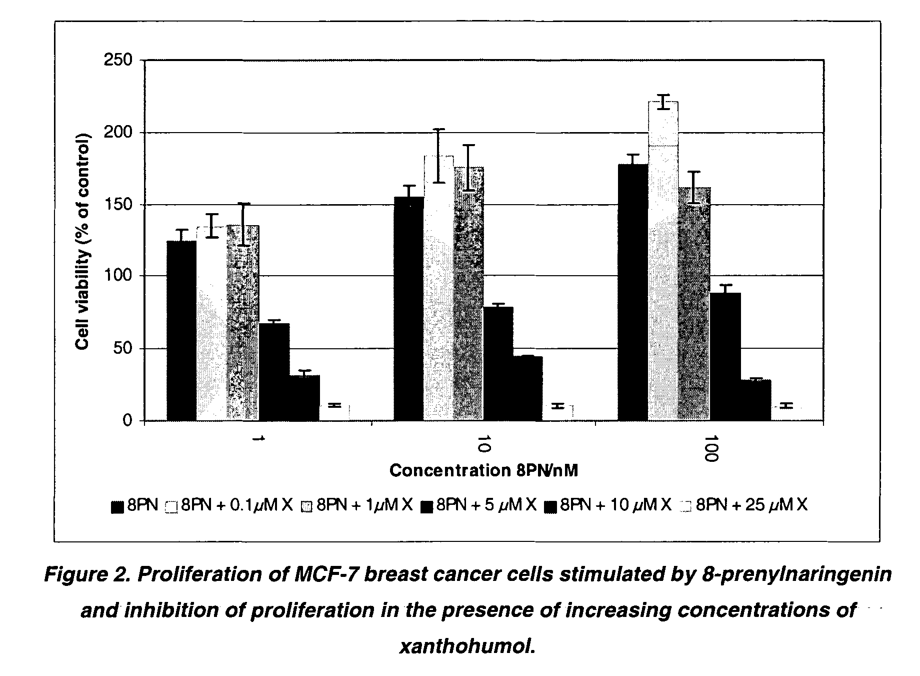

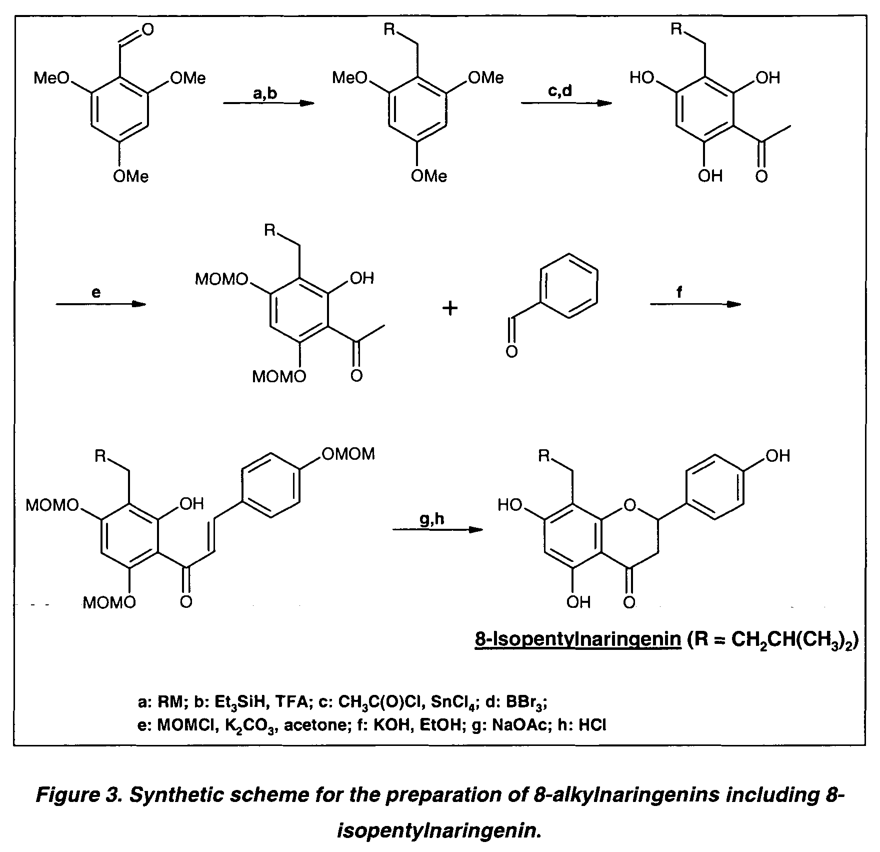

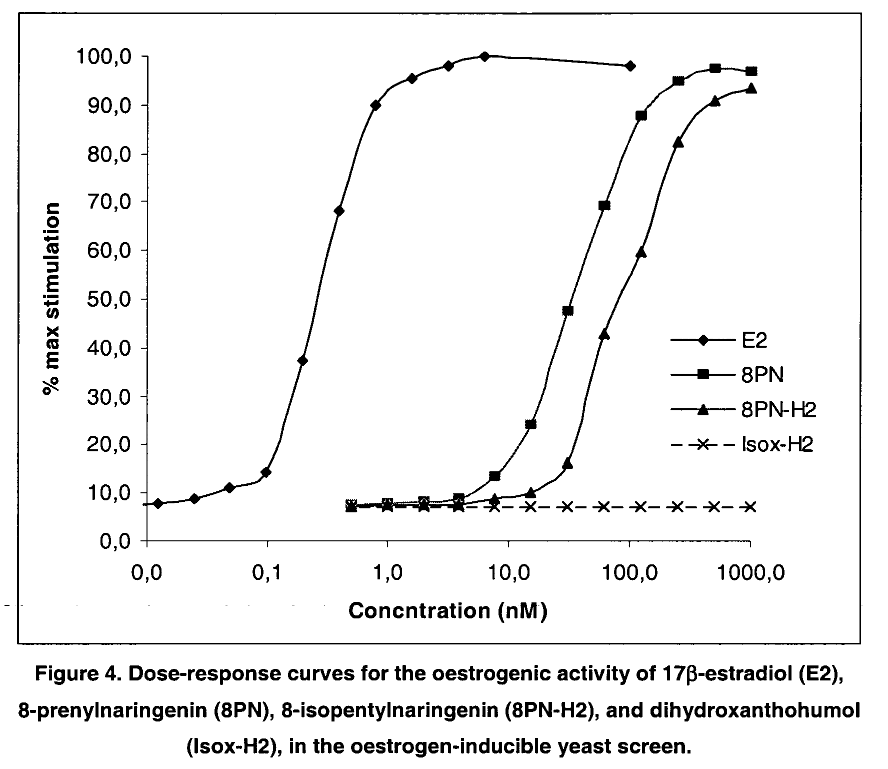

## Claims

1. A method for the production of a hop extract enriched in prenylated flavonoids or derivatives thereof, **characterised in that** the method comprises the steps of subjecting a hop product to an isomerisation reaction in the presence of an amount of a base and to at least one extraction.

2. The method according to claim 1, **characterised in that** the isomerisation reaction is carried out in alkaline conditions corresponding to concentrations of KOH (w/v %) of at least 0.1, preferably at least 0.5, more preferably of at least 1, most preferably of at least 5.

3. The method according to claim 1 or 2, **characterised in that**, before subjecting hops or a hop product to the at least one extraction and the isomerisation reaction, the hops or the hop product is subjected to an extraction in the presence of liquid or supercritical CO₂ or at least one substantially non-polar organic solvent, followed by recovering the residue containing the extract enriched in prenylated flavonoids.

4. The method according to any one of claims 1-3, **characterised in that** the method further comprises the mixing of an amount of the hop extract obtained by the at least one extraction and the isomerisation reaction as claimed in any one of claims 1-3 with an amount of a hop extract enriched in xanthohumol.

5. The method according to any one of claims 1-4, **characterised in that**, as a hop product use is made of a hop product that has been subjected to an additional extraction step with water and/or at least one non-polar organic solvent, followed by recovering the residue containing the extract enriched in prenylated flavonoids.

6. The method according to any one of claims 1-5, **characterised in that** the at least one extraction is carried out with at least one organic solvent chosen from the group of alcohols, water-based alcohols, ketones, water-based ketones or esters or mixtures thereof or alkaline water.

7. The method according to any one of claims 1-6, **characterised in that** the isomerisation reaction is carried out at a temperature between room temperature and boiling temperature of the reaction mixture.

8. The method according to any one of claims 1-7, **characterised in that** the isomerisation reaction is carried out for a time period between 0.25 and 2 h.

9. The method according to any one of claims 1-8, **characterised in that** the method further comprises the step of mixing an amount of the hop extract obtained by the at least one extraction and the isomerisation reaction as claimed in any one of claims 1-8 with an amount of a hop extract enriched in a 8-alkylnaringenin, preferably 8-isopentylnaringenin.

10. The method according to claim 9, **characterised in that** the hop extract enriched in 8-isopentylnaringenin, obtained with a method comprising the steps of:
(a) subjecting a hop extract enriched in xanthohumol to an isomerisation reaction to convert xanthohumol to isoxanthohumol; preferably the isomerisation reaction is carried out in alkaline conditions
(b) subjecting the extract obtained in step (a) to a catalytic hydrogenation reaction to convert isoxanthohumol to dihydroisoxanthohumol;
(c) subjecting the extract obtained in step (b) to a demethylation reaction to convert dihydroisoxanthohumol to 8-isopentylnaringenin.

11. The method according to claim 9, **characterised in that** the hop extract enriched in 8-alkylnaringenin is obtained by addition of an amount of a synthetic 8-alkylnaringenin, preferably an amount of synthetic 8-isopentylnaringenin.

12. A hop extract comprising a mixture of xanthohumol and 8-prenylnaringenin, whereby the weight ratio of 8-prenylnaringenin to xanthohumol is at least 10, preferably at least 20, more preferably at least 30.

13. The hop extract according to claim 11, **characterised in that** the hop extract comprises at least 0.025% (w/v) 8-prenylnaringenin and at least 2.5% (w/v) xanthohumol.

14. The hop extract according to claim 11 or 12, **characterised in that** the hop extract further comprises isoxanthohumol as a pro-oestrogen and 6-prenylnaringenin, the ratio (8-prenylnaringenin x 100%)/(8-prenylnaringenin + 6-prenylnaringenin) being at least 25%, preferably at least 50%, more preferably at least 75%.

15. The hop extract according to any one of claims 12-14, **characterised in that** the hop extract further comprises an amount of 8-alkylnaringenin, preferably 8- isopentylnaringenin.

16. Use of the hop extract according to any one of claims 12-15 or the hop extract obtainable with the method of any one of claims 1-10 for the manufacture of a medicament or a phytopharmaceutical having a combined oestrogenic and cancer-chemopreventive action.

17. Use of the hop extract according to claim 16, whereby the cancer-chemopreventive action is an antiproliferative action.

18. Use of the hop extract according to any one of claims 12-15 or the hop extract obtainable with the method of any one of claims 1-11 for the manufacture of a medicament or a phytopharmaceutical product for the treatment or prophylaxis of any one of conditions, symptoms, complaints or disease states or a combination thereof caused by a disturbance in hormonal balance of oestrogenic nature.

19. Use of the hop extract according to claim 18, whereby the condition, symptom, complaint or disease state caused by the disturbance in hormonal balance of oestrogenic nature is the menopause.

20. Use of the hop extract according to claim 18, whereby the disease state is osteoporosis.

21. Use of the hop extract according to claim 18, whereby the disease state is selected from the group consisting of Alzheimer's disease, sex hormone-dependent cancers, cardiovascular diseases, diabetes, inflammatory diseases, depression, prostate dysfunction, leukemia, inflammatory bowel disease, colon cancer.

22. A nutritional composition/supplement comprising an amount of the hop extract according to any one of claims 12-15 or an amount of the hop extract obtainable with the method of any one of claims 1-11.

23. A cosmetic composition comprising an amount of the hop extract according to any one of claims 12-15 or an amount of the hop extract obtainable with the method of any one of claims 1-11.
